# FASCICULE DE BREVET EUROPEEN

(11) **EP 1 800 657 B1**
(45) Date de publication et mention de la délivrance du brevet: **29.08.2012**
(21) Numéro de dépôt: 06291939.4
(22) Date de dépôt: 15.12.2006
(51) Int. Cl.: A61K 8/81, A61K 8/46, A61Q 5/04

(54) **Procédé de déformation permanente des cheveux comprenant l'utilisation d'un polymère fixant précipité.**
Verfahren zur dauerhaften Verformung von Haaren unter Verwendung eines gefällten fixierenden Polymers.
Permanent hair-waving process using a fixative polymer precipitate.

(30) Priorité: 20.12.2005 FR 0512970
(43) Date de publication de la demande: 27.06.2007
(73) Titulaire: L'Oréal, 75008 Paris (FR)
(72) Inventeur: Campain, Catherine, 75019 Paris (FR); Devin-Baudouin, Priscille, 69330 MEYZIEU (FR)
(74) Mandataire: Dossmann, Gérard

(56) Documents cités:
- EP-A- 1 506 768
- WO-A-99/18922
- FR-A- 2 773 071
- GB-A- 1 603 321

## Description

La présente invention se rapporte à un procédé de déformation permanente des fibres kératiniques comprenant l'utilisation d'un polymère fixant précipité.

Les procédés classiques pour mettre en forme, de façon permanente, les fibres kératiniques telles que les cheveux comprennent une étape de réduction -qui consiste à traiter les fibres kératiniques avec un agent réducteur capable de couper les ponts disulfure de la kératine- suivie d'un étape de fixation -qui consiste à traiter les fibres kératiniques avec un agent oxydant rétablissant les ponts disulfure. Ces étapes sont effectuées en maintenant les fibres kératiniques sous une contrainte mécanique, par exemple au moyen de bigoudis.

La traction exercée par les bigoudis sur les cheveux permet d'obtenir un décollement de racines, mais implique également l'ondulation des cheveux. Or, beaucoup de femmes souhaitent augmenter le volume de leur chevelure sans pour autant avoir des cheveux frisés.

On peut, certes, utiliser des bigoudis de grande taille pour atténuer l'aspect frisé et obtenir simplement une légère ondulation, mais la tenue de telles déformations permanentes n'est pas très bonne.

Un autre inconvénient des techniques de déformation permanente usuelles réside dans la sensibilisation des cheveux résultant du traitement répété des cheveux par des agents réducteurs et oxydants forts utilisés en quantités importantes.

Pour remédier à cet inconvénient, il a déjà été proposé d'utiliser des compositions réductrices gélifiées permettant de localiser le produit au niveau des racines et d'éviter ainsi une dégradation chimique du cheveu sur toute sa longueur. L'utilisation d'une composition réductrice gélifiée ne dispense toutefois pas de l'étape d'enroulement des cheveux sur des bigoudis. Cette technique reste par conséquent longue et difficile à mettre en oeuvre par l'utilisateur lui-même et donne un résultat de déformation peu durable.

Il existe un certain nombre de procédés visant à obtenir une déformation permanente des cheveux sans utilisation de moyens mécaniques de mise en forme tels que des bigoudis. Ainsi, la demande WO99/18922 divulgue un procédé de déformation permanente des cheveux, sans application d'une contrainte de tension, qui met en oeuvre une composition réductrice épaissie ayant un pouvoir collant modéré, puis une composition oxydante, sans rinçage intermédiaire destiné à éliminer l'agent réducteur.

La demande EP 1 064 921 décrit un procédé de déformation permanente des cheveux dans lequel, avant d'appliquer la composition réductrice, on applique sur le cheveux une composition séparée et distincte comprenant au moins un polymère anionique.

La demande WO01/95868 divulgue un procédé de déformation permanente des cheveux limitée à certaines zones, par exemple celle proche des racines, n'utilisant pas de moyens mécaniques de mise sous tension des cheveux. Le maintien de la forme imposée aux cheveux pendant les phases de réduction et d'oxydation est assuré par la composition réductrice elle-même qui contient une cire, des particules hydrophiles absorbantes, en particulier des particules de farine, et un agent réducteur absorbé par les particules hydrophiles. Ce procédé est relativement complexe, car il implique la fusion de la cire dans un dispositif de chauffage approprié et le mélange de la cire fondue avec les particules hydrophiles absorbantes et l'agent réducteur. L'application du mélange chaud sur les cheveux demande au coiffeur une certaine dextérité et est difficile à réaliser par l'utilisateur lui-même. Par ailleurs, on observe un décapage de la couleur relativement important lorsque cette technique est utilisée pour la déformation permanente de cheveux colorés.

La demande FR 2 858 551 divulgue un procédé de déformation permanente des cheveux, sensiblement plus facile à mettre en oeuvre que celui décrit dans demande WO01/95868, et qui ne nécessite pas une préparation préalable de la composition réductrice au moyen d'un dispositif de chauffage. Le procédé décrit dans la demande FR 2 858 551 comprend dans l'ordre, les étapes suivantes :
(a) application, sur les zones à déformer, d'une solution d'un polymère anionique ou amphotère dans un milieu contenant de l'eau et, éventuellement, un ou plusieurs solvants cosmétiquement acceptables,
(b) application, sur les zones traitées dans l'étape (a), d'une composition réductrice contenant un agent réducteur capable de couper les ponts disulfure de la kératine et une quantité suffisante de cations métalliques et/ou d'ions ammonium pour faire précipiter *in situ* le polymère anionique ou amphotère,
(c) mise en forme des cheveux ainsi traités suivie d'un temps de pause de la composition réductrice,
(d) application, sur les zones traitées dans les étapes (a) et (b), d'une composition fixatrice contenant un agent oxydant, suivie d'un temps de pause de la composition oxydante, et
(e) rinçage des cheveux.

Il a été observé que l'effet obtenu au moyen de ce procédé n'a une durée que limitée : la tenue de la mise en forme des cheveux notamment face aux lavages répétés n'est pas complètement satisfaisante. De plus la quantité de polymère fixant requise pour la mise en oeuvre de ce procédé rend ce procédé coûteux.

Le problème posé dans la présente demande est l'obtention d'un procédé de déformation permanente des fibres kératiniques simple à mettre en oeuvre -notamment qui ne nécessite pas l'utilisation de moyens mécaniques de mise sous tension-, qui permette d'obtenir une mise en forme durable des fibres kératiniques et qui génère une sensibilisation moindre des fibres kératiniques qu'une permanente classique.

De manière avantageuse et inattendue, la Demanderesse vient maintenant de découvrir qu'il est possible de résoudre ce problème par la mise en oeuvre du procédé de déformation permanente des fibres kératiniques selon la présente demande.

Le procédé de déformation permanente des fibres kératiniques, de préférence les fibres kératiniques humaines et en particulier les cheveux, comprend les étapes suivantes, dans l'ordre suivant :
(a) application sur les fibres kératiniques d'une composition réductrice (A) contenant un agent réducteur des fibres kératiniques et au moins un sel organique ou minéral,
(b) mise en forme des fibres kératiniques par coiffage et application sur les fibres kératiniques d'une composition (B) comprenant au moins un polymère fixant choisi parmi les polymères fixant anioniques et amphotères, dans un milieu aqueux, alcoolique ou hydro-alcoolique,
(c) après un temps de pause, application sur les fibres kératiniques d'une composition fixatrice (C) contenant un agent oxydant,
(d) après un temps de pause, rinçage des fibres kératiniques ;
les sous-étapes de mise en forme des fibres kératiniques par coiffage et application sur les fibres kératiniques d'une composition (B) de l'étape (b) étant réalisées dans un des ordres suivants : mise en forme des fibres kératiniques par coiffage puis application sur les fibres kératiniques d'une composition (B) ; application sur les fibres kératiniques d'une composition (B) puis mise en forme des fibres kératiniques par coiffage ; ou mise en forme des fibres kératiniques par coiffage et simultanément application sur les fibres kératiniques d'une composition (B).

Dans une première variante de ce procédé, les sous-étapes de l'étape (b) sont réalisées dans l'ordre suivant : mise en forme des fibres kératiniques par coiffage puis application sur les fibres kératiniques d'une composition (B).

Dans une deuxième variante de ce procédé, les sous-étapes de l'étape (b) sont réalisées dans l'ordre suivant : application sur les fibres kératiniques d'une composition (B) puis mise en forme des fibres kératiniques par coiffage.

Dans une troisième variante de ce procédé les sous-étapes de l'étape (b) sont réalisées simultanément.

De manière préférée, l'étape (b) est suivie d'un rinçage.

Ce procédé présente également l'avantage d'être sélectif, c'est-à-dire qu'il peut être mis en oeuvre sur une partie des cheveux : seulement sur une zone de la tête par exemple pour corriger un épi, ou seulement sur une partie du cheveu par exemple au niveau des racines.

Le procédé selon la présente demande permet une mise en forme des cheveux qui dure plus longtemps que la mise en forme obtenue avec le procédé divulgué dans la demande FR 2 858 551, sans utilisation d'une quantité importante de polymère fixant. Un autre intérêt du procédé selon la présente demande est son avantage économique.

D'autres caractéristiques, aspects, objets et avantages de la présente invention apparaîtront encore plus clairement à la lecture de la description et des exemples qui suivent.

La composition réductrice (A) contient un agent réducteur des fibres kératiniques et au moins un sel organique ou minéral, les sels étant de préférence utilisés en une quantité apte à précipiter au moins une partie et de préférence tout le polymère fixant contenu dans la composition (B).

Par « polymère fixant dans un milieu aqueux, alcoolique ou hydro-alcoolique », tels que les polymères utilisés dans la composition (B), on entend que ce polymère est en solution, en émulsion ou en dispersion dans ledit milieu aqueux, alcoolique ou hydro-alcoolique utilisé.

Sans vouloir être liée par une quelconque théorie, la Demanderesse pense que lors de l'application de la composition (B) comprenant le polymère fixant, le mélange -sur les fibres kératiniques- de la composition réductrice fortement chargée en sels organiques ou minéraux et de la composition comprenant des polymères fixants permet une forte augmentation de la force ionique du milieu. Il se produit alors une contraction de la pelote macromoléculaire aboutissant au final à la précipitation du polymère fixant. Il se produit alors un durcissement de la fibre ou de la partie de fibre sur laquelle le polymère a été déposé, ce durcissement permet de maintenir les cheveux dans la forme voulue pendant les étapes de réduction et de fixation.

La force ionique nécessaire pour obtenir la précipitation *in situ* du polymère dépend d'un certain nombre de facteurs tels que le nombre de charges du polymère, le caractère plus ou moins hydrophobe du polymère sous sa forme non chargée, la masse moléculaire du polymère, la concentration du polymère fixant dans la composition (B) ou encore le pH du milieu obtenu par mélange *in situ* des compositions (A) et (B).

Cette force ionique peut être amenée par des sels minéraux ou organiques présents dans la composition réductrice (A), mais également par l'agent réducteur lui même lorsqu'il est sous forme de sel.

Pour augmenter la force ionique du milieu de réduction jusqu'à une valeur suffisante, on peut utiliser en principe n'importe quel sel organique ou minéral qui n'interfère pas de manière significative avec la réaction de réduction des ponts disulfure. On préfère en particulier les sels d'ammonium et les sels de métaux alcalins.

Lorsque les cations sont apportés par l'agent réducteur (sous forme de sel) celui-ci comporte une ou plusieurs fonctions carboxylate. On peut citer à titre d'exemple le thioglycolate d'ammonium ou le thiolactate d'ammonium.

De préférence, les sels sont des sels de métaux alcalins, de métaux alcalino-terreux ou d'ammonium. A titre d'exemples de sels minéraux utilisables, on peut citer les halogénures de métaux alcalins ou alcalino-terreux tels que le chlorure de sodium ou le chlorure de magnésium, et les carbonates tels que le carbonate d'ammonium. Les sels organiques peuvent être par exemple le citrate d'ammonium.

L'étape de réduction du procédé de la présente invention est de préférence mise en oeuvre à un pH compris entre 7 et 11, en particulier entre 7,5 et 9,5.

L'homme du métier saura choisir, grâce à ses connaissances techniques générales, les agents d'ajustement du pH et agents tampon appropriés pour ajuster le pH des compositions (A) et (B) à une valeur adaptée.

La Demanderesse a constaté qu'une concentration en sels organiques ou minéraux dans la composition réductrice appliquée (composition (A)) supérieure à 0,3 M (mol/l) était généralement suffisante pour obtenir un dépôt de polymères précipités d'une qualité satisfaisante permettant le maintien de la forme de la chevelure pendant les étapes de coiffage et d'oxydation. De préférence, cette concentration est comprise entre 0,5 et 6 M.

L'agent réducteur de la composition réductrice peut être n'importe quel agent réducteur de la kératine connu, choisi parmi ceux utilisés habituellement dans le domaine de la déformation permanente des cheveux, tels que les sulfites, les bisulfites et les thiols. Parmi ces agents réducteurs, on préfère en particulier la cystéine, la cystéamine, l'acide thiolactique et l'acide thioglycolique et les sels cosmétiquement acceptables de ceux-ci, en particulier le thioglycolate d'ammonium.

La concentration de cet agent réducteur dans la composition réductrice va de 1 % à 25 % en poids, de préférence de 1 à 10 % en poids.

On peut laisser agir l'agent réducteur pendant un temps suffisant pour obtenir la réduction souhaitée. Ce temps de pose est alors de préférence compris entre 2 et 30 minutes, et en particulier entre 5 et 15 minutes.

De préférence, on met en forme directement la chevelure sans laisser pauser le réducteur.

Pour la mise en oeuvre de la composition (B), on peut, en principe, utiliser n'importe quel polymère fixant naturel ou synthétique permettant de conférer une forme à la chevelure ou de modifier la forme de ladite chevelure. De préférence le polymère fixant utilisé est anionique ou amphotère ; il peut aussi s'agir de mélanges de polymères anioniques et amphotères.

Les polymères anioniques généralement utilisés dans la composition (B), sont des polymères comportant des groupes dérivés d'acides carboxylique, sulfonique ou phosphorique, et présentant une masse moléculaire en poids comprise entre 500 et 5 000 000.

Les groupements carboxyliques sont apportés par des monomères monoacides ou diacides carboxyliques insaturés tels que ceux répondant à la formule : 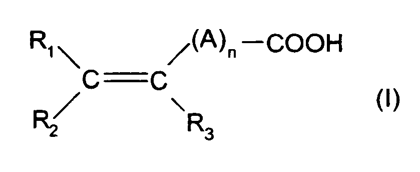 dans laquelle n est un nombre entier de 0 à 10, A désigne un groupement méthylène, éventuellement relié à l'atome de carbone du groupement insaturé ou au groupement méthylène voisin lorsque n est supérieur à 1, par l'intermédiaire d'un hétéroatome tel que l'oxygène
ou le soufre, R₁ désigne un atome d'hydrogène, un groupement phényle
ou benzyle, R₂ désigne un atome d'hydrogène, un groupement alkyle inférieur ou carboxyle, R₃ désigne un atome d'hydrogène, un groupement alkyle inférieur, un groupement -CH₂-COOH, phényle ou benzyle.

Dans la formule (I) ci-dessus, un groupement alkyle inférieur comporte de préférence de 1 à 4 atomes de carbone et désigne en particulier, les groupements méthyle et éthyle.

Les polymères anioniques à groupements carboxyliques ou sulfoniques préférés sont :

A) les homo- ou copolymères d'acide acrylique ou méthacrylique ou leurs sels, dont les copolymères d'acide acrylique et d'acrylamide et les copolymères acide méthacrylique/acide acrylique/acrylate d'éthyle/méthacrylate de méthyle, en particulier l'AMERHOLD DR 25 commercialisé par la société AMERCHOL, et les sels de sodium des acides polyhydroxycarboxyliques. On peut citer également les copolymères acide méthacrylique/acrylate d'éthyle, notamment en dispersion aqueuse, tels que les LUVIFLEX SOFT et LUVIMER MAE commercialisés par la société BASF.

B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois 75370 et 75371. On peut également citer les copolymères d'acide acrylique et de méthacrylate d'alkyle en C₁-C₄.

C) les copolymères dérivés d'acide crotonique tels que ceux comportant dans leur chaîne des motifs acétate ou propionate de vinyle, et éventuellement d'autres monomères tels que les ester allylique ou méthallylique, éther vinylique ou ester vinylique d'un acide carboxylique saturé, linéaire ou ramifié, à longue chaîne hydrocarbonée, tels que ceux comportant au moins 5 atomes de carbone, ces polymères pouvant éventuellement être greffés et réticulés, ou encore un ester vinylique, allylique ou méthallylique d'un acide carboxylique α ou β-cyclique. De tels polymères sont décrits entre autres dans les brevets français numéros 1 222 944, 1 580 545, 2 265 782, 2 265 781, 1 564 110 et 2 439 798. Des produits commerciaux entrant dans cette classe sont les résines 28-29-30, 26-13-14 et 28-13-10 vendues par la société NATIONAL STARCH.

On peut citer aussi comme copolymère dérivé d'acide crotonique les terpolymères acide crotonique/acétate de vinyle/t.butylbenzoate de vinyle et en particulier le MEXOMERE PW fourni par la société CHIMEX.

D) Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2 047 398, 2 723 248, 2 102 113, le brevet GB 839 805, et notamment ceux vendus sous les dénominations GANTREZ® AN ou ES par la société ISP.

Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acryliques ou méthacryliques ou la vinylpyrrolidone dans leur chaîne, les fonctions anhydrides sont monoestérifiées ou monoamidifiées. Ces polymères sont par exemple décrits dans les brevets français 2 350 384 et 2 357 241 de la demanderesse.

E) Les polyacrylamides comportant des groupements carboxylates.

F) Les polymères comprenant les groupements sulfoniques. Ces polymères peuvent être des polymères comportant des motifs vinylsulfonique, styrène-sulfonique, naphtalène-sulfonique, acrylamido-alkylsulfonique, sulfoisophtalates.

Ces polymères peuvent être notamment choisis parmi :
- les sels de l'acide polyvinylsulfonique ayant une masse moléculaire comprise entre environ 1 000 et 100 000 ainsi que les copolymères avec un comonomère insaturé tel que les acides acrylique ou méthacrylique, et leurs esters, ainsi que l'acrylamide ou ses dérivés, les éthers vinyliques et la vinylpyrrolidone ;
- les sels de l'acide polystyrène-sulfonique, les sels de sodium, ayant une masse moléculaire d'environ 500 000 et d'environ 100 000. Ces composés sont décrits dans le brevet FR 2198719 ;
- les sels d'acides polyacrylamide-sulfoniques tels que ceux mentionnés dans le brevet US 4 128 631 ;

G) les polymères siliconés anioniques greffés ;

Les polymères siliconés greffés utilisés sont choisis préférentiellement parmi les polymères à squelette organique non-siliconé greffé par des monomères contenant un polysiloxane, les polymères à squelette polysiloxanique greffé par des monomères organiques non-siliconés et leurs mélanges.

Dans tout ce qui suit ou qui précède, on entend désigner par silicone ou polysiloxane, en conformité avec l'acceptation générale, tout polymère oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁-C₁₀ et en particulier méthyle, les radicaux fluoralkyles, les radicaux aryles et en particulier phényle, et les radicaux alcényles et en particulier vinyle ; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyles ou hydroxyalkyles, les groupements aminés substitués ou non, les groupements amides, les radicaux acyloxy ou acyloxyalkyles, les radicaux hydroxyalkylamino ou aminoalkyles, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites « organomodifiées »).

Dans ce qui suit, on entend désigner par « macromère polysiloxane » en conformité avec l'acceptation générale, tout monomère contenant dans sa structure une chaîne polymère du type polysiloxane.

Les polymères à squelette organique non-siliconé greffé par des monomères contenant un polysiloxane utilisé selon la présente invention sont constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromère polysiloxane.

Les monomères organiques non-siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture de cycle tels que ceux du type oxazoline ou caprolactone.

Les polymères à squelette organique non-siliconé greffé par des monomères contenant un polysiloxane utilisables peuvent être obtenus selon tout moyen connu de l'homme de l'art, en particulier par réaction entre (i) un macromère polysiloxane de départ correctement fonctionnalisé sur la chaîne polysiloxanique et (ii) un ou plusieurs composés organiques non-siliconés, eux-mêmes correctement fonctionnalisés par une fonction qui est capable de venir réagir avec le ou les groupements fonctionnels portés par ladite silicone en formant une liaison covalente ; un exemple classique d'une telle réaction est la réaction radicalaire entre un groupement vinylique porté sur une des extrémités de la silicone avec une double liaison d'un monomère à insaturation éthylénique de la chaîne principale.

Les polymères à squelette organique non-siliconé greffé par des monomères contenant un polysiloxane, conformes à l'invention, sont choisis plus préférentiellement parmi ceux décrits dans les brevets US 4,693,935, US 4,728,571 et US 4,972,037 et les demandes de brevet EP-A-0412704, EP-A-0412707, EP-A-0640105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Une famille particulière de polymères siliconés greffés convenant pour la réalisation de la présente invention est constituée par les copolymères greffés siliconés comprenant :
a) de 0 à 98 % en poids d'au moins un monomère (A) lipophile de faible polarité lipophile à insaturation éthylénique, polymérisable par voie radicalaire ;
b) de 1 à 98 % en poids d'au moins un monomère (B) hydrophile polaire à insaturation éthylénique, copolymérisable avec le ou les monomères du type (A) ;
c) de 0,01 à 50 % en poids d'au moins un macromère polysiloxane (C) de formule générale :

   X(Y)ₙSi(R)₃₋ₘZₘ (II)

   Où :
   X désigne un groupe vinylique copolymérisable avec les monomères (A) et (B) ;
   Y désigne un groupe de liaison divalent ;
   R désigne un hydrogène, un alkyle ou un alcoxy en C₁-C₆, un aryle C₆-C₁₂ ;
   Z désigne un motif polysiloxane monovalent ayant un poids moléculaire moyen en nombre d'au moins 500 ;
   n est 0 ou 1 et m est un entier allant de 1 à 3 ; les pourcentages étant calculés par rapport au poids total des monomères (A), (B) et (C).

Ces polymères sont décrits ainsi que leurs procédés de préparation dans les brevets US 4,963,935, US 4,728,571 et US 4,972,037 et les demandes de brevet EP-A-0412704, EP-A-0412707, EP-A-0640105. Ils ont un poids moléculaire moyen en nombre de préférence allant de 10 000 à 2 000 000 et de préférence une température de transition vitreuse Tg ou une température de fusion cristalline Tm d'au moins -20°C.

On peut citer comme exemples de monomères lipophiles (A), les esters d'acide acrylique ou méthacrylique d'alcools en C₁-C₁₈ ; le styrène ; les macromères polystyrène ; l'acétate de vinyle ; le propionate de vinyle ; l'alpha-méthylstyrène ; le tertio-butylstyrène ; le butadiène ; le cyclohexadiène ; l'éthylène ; le propylène ; le vinyltoluène ; les esters d'acide acrylique ou méthacrylique et de 1,1-dihydroperfluoroalcanol ou de ses homologues ; les esters d'acide acrylique ou méthacrylique et de oméga-hydrydofluoroalcanol ; les esters d'acide acrylique ou méthacrylique et de fluoroalkylsulfoamido-alcool ; les esters d'acide acrylique ou méthacrylique et d'alcool fluoroalkylique ; les esters d'acide acrylique ou méthacrylique et de fluoréther d'alcool ; ou leurs mélanges.

Les monomères (A) préférentiels sont choisis dans le groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, le 2-(N-méthyl perfluoroctane sulfoamido)éthylacrylate ; le 2-(N-butylperflurooctane sulfoamido)éthylacrylate et leurs mélanges.

On peut citer comme exemples de monomères polaires (B), l'acide acrylique, l'acide méthacrylique, le N,N-diméthylacrylamide, le méthacrylate de diméthylaminoéthyle, le méthacrylate de diméthylaminoéthyle quaternisé, le (méth)acrylamide, le N-t-butylacrylamide, l'acide maléique, l'anhydride maléique et leurs demi-esters, les (méth)acrylates hydroxyalkylés, le chlorure de diallyldiméthylammonium, la vinylpyrrolidone, les éthers de vinyle, les maléimides, la vinylpyridine, le vinylimidazole, les composés polaires vinyliques hétérocycliques, le styrène sulfonate, l'alcool allylique, l'alcool vinylique, le vinylcaprolactame ou leurs mélanges. Les monomères (B) préférentiels sont choisis dans le groupe constitué par l'acide acrylique, le N,N-diméthylacrylamide, le méthacrylate de diméthylaminoéthyle, le méthacrylate de diméthylaminoéthyle quaternisé, la vinylpyrrolidone et leurs mélanges.

A titre de monomères polaires (B), les polymères siliconés greffés anioniques utilisés selon l'invention contiennent au moins un monomère anionique.

Les macromères polysiloxane (C) de formule (I) préférentiels sont choisis parmi ceux répondant à la formule générale suivante (III) : 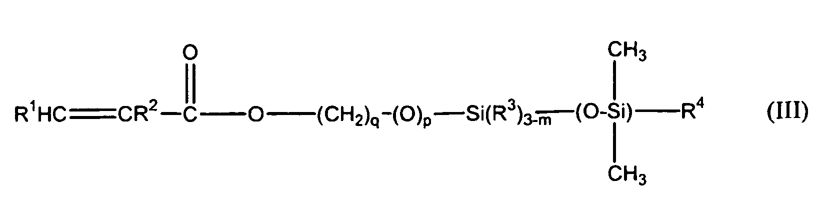 dans laquelle :
R¹ est l'hydrogène ou -COOH (de préférence hydrogène) ;
R² est l'hydrogène, méthyle ou -CH₂COOH (de préférence méthyle) ;
R³ est un groupe alkyle, alcoxy ou alkylamino en C₁-C₆, aryle en C₁-C₁₂ ou hydroxyle (de préférence méthyle) ;
R⁴ est un groupe alkyle, alcoxy ou alkylamino en C₁-C₆, aryle en C₁-C₁₂ ou hydroxyle (de préférence méthyle) ;
q est un entier de 2 à 6 (de préférence 3) ;
p est 0 ou 1 ;
r est un nombre entier de 5 à 700 ;
m est un entier allant de 1 à 3 (de préférence 1) ;

On utilise plus particulièrement les macromères polysiloxanes de formule : 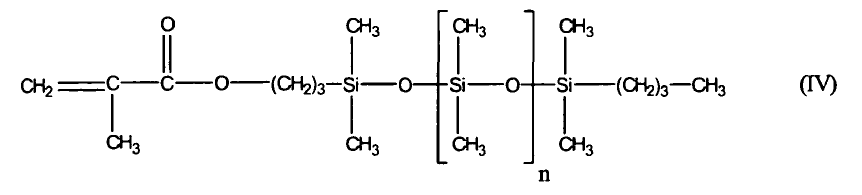 n étant un entier allant de 5 à 700.

Un mode particulier de réalisation de l'invention consiste à utiliser un copolymère susceptible d'être obtenu par polymérisation radicalaire à partir du mélange de monomères constitué par :
a) 60 % en poids d'acrylate de tertiobutyle :
b) 20 % en poids d'acide acrylique ;
c) 20 % en poids de macromère siliconé de formule : 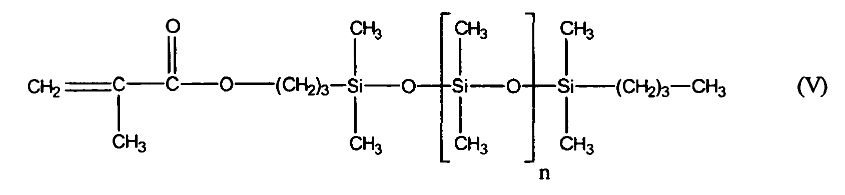 n étant un entier allant de 5 à 700 ; les pourcentages en poids étant calculés par rapport au poids total des monomères.

Une autre famille particulière de polymères siliconés convenant pour la réalisation de la présente invention est constituée par les copolymères greffés siliconés susceptibles d'être obtenus par extrusion réactive d'un macromère polysiloxane ayant une fonction réactive terminale sur un polymère du type polyoléfine comportant des groupes réactifs susceptibles de réagir avec la fonction terminale du macromère polysiloxane pour former une liaison covalente permettant le greffage de la silicone sur la chaîne principale de la polyoléfine.

Ces polymères sont décrits ainsi que leur procédé de préparation dans la demande de brevet WO 95/00578.

Les polyoléfines réactives sont choisies de préférence parmi les polyéthylènes ou les polymères de monomères dérivés de l'éthylène tels que le propylène, le styrène, les alkylstyrènes, le butylène, le butadiène, les (méth)acrylates, les esters de vinyle ou équivalents, comportant des fonctions réactives susceptibles de réagir avec la fonction terminale du macromère polysiloxane. Ils sont choisis plus particulièrement parmi les copolymères d'éthylène ou de dérivés d'éthylène et de monomères choisis parmi ceux comportant une fonction carboxylique tels que l'acide (méth)acrylique ; ceux comportant une fonction anhydride d'acide tels que l'anhydride de l'acide maléique ; ceux comportant une fonction chlorure d'acide tels que le chlorure de l'acide (méth)acrylique ; ceux comportant une fonction ester tels que les esters de l'acide (méth)acrylique ; ceux comportant une fonction isocyanate.

Les macromères siliconés sont choisis de préférence parmi les polysiloxanes comportant un groupe fonctionnalisé, en bout de la chaîne polysiloxanique ou à proximité de l'extrémité de ladite chaîne, choisi dans le groupe constitué par les alcools, les thiols, les époxy, les amines primaires et secondaires et plus particulièrement parmi ceux répondant à la formule générale (VI) :

T-(CH₂)ₛ-Si-[(OSiR⁵R⁶)ₜ-R⁷]_{y} (VI)

dans laquelle T est choisi dans le groupe constitué par NH₂, NHR', une fonction époxy, OH, SH ; R⁵, R⁶, R⁷ et R', indépendamment, désignent un alkyle en C₁-C₆, phényle, benzyle, ou alkylphényle en C₆-C₁₂, hydrogène ; s est un nombre de 2 à 100 ; t est un nombre de 0 à 1000 et y est un nombre de 1 à 3. Ils ont un poids moléculaire moyen en nombre de préférence allant de 5 000 à 300 000, plus préférentiellement de 8 000 à 200 000 et plus particulièrement de 9 000 à 40 000.

Selon la présente invention, le ou les polymères siliconés greffés, à squelette polysiloxanique greffé par des monomères organiques non-siliconés comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Les polymères à squelette polysiloxanique greffé par des monomères organiques non-siliconés selon l'invention peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements ≡Si-H et des groupements vinyliques CH₂=CH-, ou encore la réaction entre des groupements thio-fonctionnels-SH avec ces mêmes groupements vinyliques.

Des exemples de polymères à squelette polysiloxanique greffé par des monomères organiques non-siliconés convenant à la mise en oeuvre de la présente invention, ainsi que leur mode particulier de préparation, sont notamment décrits dans les demandes de brevets EP-A-0582152, WO 93/23009 et WO 95/03776 dont les enseignements sont totalement inclus dans la présente description à titre de références non limitatives.

Selon un mode particulièrement préféré de réalisation de la présente invention, le polymère siliconé, à squelette polysiloxanique greffé par des monomères organiques non-siliconés, mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.

Selon la présente invention, lesdits monomères anioniques à insaturation éthylénique sont de préférence choisis, seuls ou en mélanges, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous forme d'un sel.

Selon la présente invention, les monomères hydrophobes à insaturation éthylénique sont de préférence choisis, seuls ou en mélanges, parmi les esters d'acide acrylique alcanols et/ou les esters d'acide méthacrylique d'alcanols. Les alcanols sont de préférence en C₁-C₁₈ et plus particulièrement en C₁-C₁₂. Les monomères préférentiels sont choisis dans le groupe constitué par le (méth)acrylate d'isooctyle, le (méth)acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle ou leurs mélanges.

Une famille de polymères siliconés à squelette polysiloxanique greffé par des monomères organiques non-siliconés convenant particulièrement bien à la mise en oeuvre de la présente invention est constituée par les polymères siliconés comportant dans leur structure le motif de formule (VII) suivant : 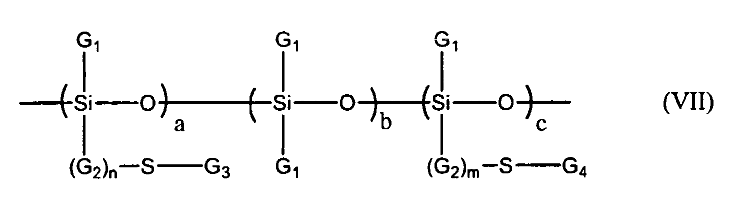 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkyle en C₁-C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (VII) ci-dessus présente au moins l'une, et encore plus préférentiellement l'ensemble, des caractéristiques suivantes :
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle(C₁-C₁₀), de préférence le (méth)acrylate d'isobutyle ou de méthyle.

Des exemples de polymères siliconés greffés répondant à la formule (IV) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.

De préférence, la masse moléculaire en nombre des polymères siliconés à squelette polysiloxanique greffé par des monomères organiques non-siliconés de l'invention varie de 10 000 à 1 000 000 environ, et encore plus préférentiellement de 10 000 à 100 000 environ.

A titre de polymères siliconés greffés utilisables selon la présente invention, on peut citer le produit commercialisé par la société 3M sous la référence VS80.

H) Les polyuréthanes anioniques.

Les polyuréthanes utilisés de préférence selon l'invention présentent de préférence un motif répétitif de base répondant à la formule (VIII) suivante :

-X'-B-X'-CO-NH-R-NH-CO- (VIII)

dans laquelle
- X' représente O et/ou NH,
- B est un radical hydrocarboné divalent, ce radical étant substitué ou non, et
- R est un radical divalent choisi parmi les radicaux alkylènes ramifiés ou non de type aromatique en C₆-C₂₀, aliphatique en C₁ à C₂₀, de préférence en C₁-C₆, cycloaliphatique en C₁ à C₂, de préférence en C₁-C₆, ces radicaux étant non substitués ou substitués par un ou plusieurs groupements halogène, alcoxy en C₁-C₄, aryle en C₆-C₃₀ en particulier phényle.

De préférence, le radical B est un radical divalent en C₁-C₃₀, de préférence C₂-C₁₀ et est porteur d'un groupement présentant une ou des fonctions carboxyliques et/ou une ou des fonctions sulfoniques, lesdites fonctions carboxyliques et/ou sulfoniques étant sous forme libre ou partiellement ou totalement neutralisées par une base minérale ou organique telle que les hydroxydes des métaux alcalins ou alcalinoterreux, l'ammoniaque et les alkylamines ou alcanolamines, les acides aminés organiques. De préférence B est le radical divalent issu de l'acide diméthylolpropionique.

Le radical R est avantageusement choisi parmi les radicaux répondant aux formules suivantes : 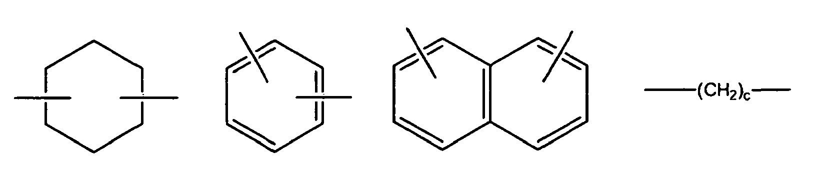 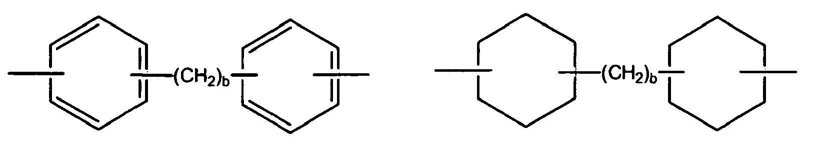 dans lesquelles b est un nombre entier compris entre 0 et 3, et c un nombre entier compris entre 1 et 20, de préférence compris entre 2 et 12.

En particulier, le radical R est choisi parmi les radicaux hexaméthylène, 4,4'-biphénylèneméthane, 2,4 et/ou 2,6-tolylène, 1,5-naphtylène, p-phénylène, méthylène-4,4bis-cyclohexyle et le radical divalent dérivé de l'isophorone.

Les polyuréthanes fixant utilisables peuvent comporter des greffons silicones et des silicones à greffons hydrocarbonés.

Un polyuréthane utilisable peut avantageusement comprendre en outre au moins une séquence polysiloxane et son motif répétitif de base répond par exemple à la formule générale (IX) :

-X'-P-X'-CO-NH-R-NH-CO- (IX)

dans laquelle :
- P est un segment polysiloxanique,
- X' représente O et/ou NH, et
- R est un radical divalent choisi parmi les radicaux alkylènes ramifiés ou non de type aromatique en C₆-C₂₀, aliphatique en C₁-C₂₀, de préférence en C₁-C₆, cycloaliphatique en C₁-C₂₀, de préférence en C₁-C₆, ces radicaux étant non substitués ou substitués par un ou plusieurs groupements halogène, alcoxy en C₁-C₄, aryle en C₁-C₃₀, en particulier phényle.

Le radical R est avantageusement choisi parmi les radicaux répondant aux formules suivantes : 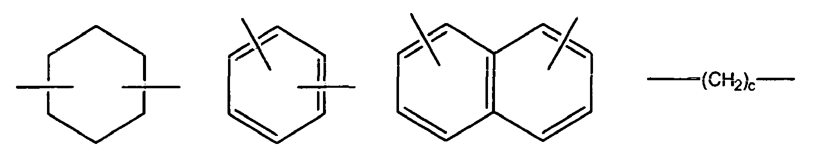 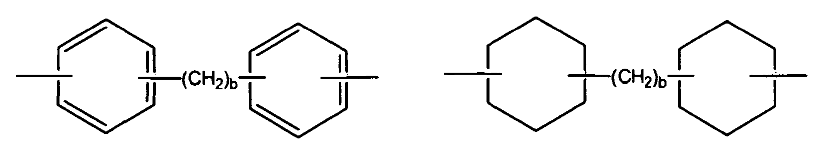 dans lesquelles b est un nombre entier compris entre 0 et 3, et c un nombre entier compris entre 1 et 20, de préférence compris entre 2 et 12.

En particulier, le radical R est choisi parmi les radicaux hexaméthylène, 4,4'-biphénylèneméthane, 2,4-et/ou 2,6-tolylène, 1,5-naphtylène, p-phénylène, méthylène-4,4bis-cyclohexyle et le radical divalent dérivé de l'isophorone.

Avantageusement, le segment polysiloxanique P répond à la formule générale (X) ci-après : 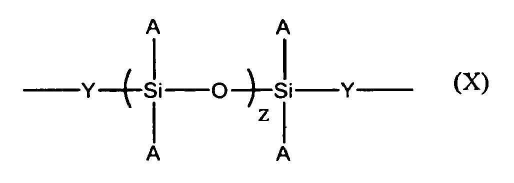 dans laquelle :
- les groupes A, qui peuvent être identiques ou différents, sont choisis parmi d'une part les groupes hydrocarbonés monovalents en C₁ à C₂₀, substantiellement exempts d'insaturation éthylénique et, d'autre part, les groupes aromatiques,
- Y représente un groupe hydrocarboné divalent, et
- Z représente un nombre entier, choisi de telle sorte que la masse moléculaire moyenne du segment polysiloxane soit comprise entre 300 et 10 000.

En général, le groupe divalent Y est choisi parmi les groupes alkylène de formule -(CH₂)a-, dans laquelle a représente un nombre entier pouvant être compris entre 1 et 10.

Les groupes A peuvent être choisis parmi les groupes alkyle en C₁-C₁₈, en particulier les groupes méthyle, éthyle, propyle, isopropyle, butyle, pentyle, hexyle, octyle, décyle, dodécyle et octadécyle ; les groupes cycloalkyle, en particulier le groupe cyclohexyle ; les groupes aryle, notamment phényle et naphtyle : les groupes arylalkyle, notamment benzyle et phényléthyle, ainsi que les groupes tolyle et xylyle.

A titre d'exemples de polyuréthane fixant, on peut notamment citer le copolymère acide diméthylolpropionique/isophorone-diisocyanate/néopentylglycol/polyesterdiols (connu aussi sous le nom de polyuréthane-1, appellation INCI) vendu sous la marque Luviset® PUR par la société BASF, le copolymère acide diméthylolpropionique/isophorone- diisocyanate/néopentylglycol/polyesterdiols/dia-mine siliconée (connu aussi sous le nom de polyuréthane-6, appellation INCI) vendu sous la marque Luviset® Si PUR A par la société BASF.

Comme autre polyuréthane anionique, on peut aussi utiliser l'AVALURE UR 450.

On peut également utiliser les polymères à groupements sulfoisophtalates, tels que les polymères AQ55 et AQ48 commercialisés par la société EASTMAN.

Selon l'invention, les polymères anioniques sont de préférence choisis parmi les copolymères d'acide acrylique tels que le terpolymère acide acrylique/acrylate d'éthyle/N-tertiobutylacrylamide vendu sous la dénomination ULTRAHOLD STRONG® par la société BASF, les copolymères acide méthacrylique/acrylate d'éthyle, notamment en dispersion aqueuse, tels que les LUVIFLEX SOFT et LUVIMER MAE commercialisés par la société BASF. Des copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléïque monoestérifié vendu sous la dénomination GANTREZ® ES 425 par la société ISP, le LUVISET SI PUR, le MEXOMERE PW, les polyuréthanes anioniques élastomères ou non, les polymères à groupements sulfoisophtalates, les polymères siliconés greffés anioniques, ainsi que l'AMERHOLD DR 25, le VS 80.

Les polymères amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes ; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus que l'on préfère plus particulièrement, sont choisis parmi les polymères suivants :

1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537.

Le composé vinylique peut être également un sel de dialkyldiallylammonium tel que le chlorure de diéthyldiallylammonium.

(2) les polymères comportant des motifs dérivant :
a) d'au moins un monomère choisi parmi les acrylamides ou les méthacrylamides substitués sur l'azote par un groupe alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.

Les acrylamides ou méthacrylamides N-substitués plus particulièrement préférés selon l'invention sont les groupements dont les groupes alkyle contiennent de 2 à 12 atomes de carbone et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique. Les comonomères basiques préférés sont des méthacrylates d'aminoéthyle, de butylaminoéthyle, de N,N'-diméthylaminoéthyle, de N-tertio-butylaminoéthyle. On utilise particulièrement les copolymères dont la dénomination CTFA (4ème Ed, 1991) est Octylacrylamide/acrylates/butylaminoethylmethacrylate copolymer tels que les produits vendus sous la dénomination AMPHOMER® ou LOVOCRYL® 47 par la société NATIONAL STARCH.

(3) les polyaminoamides réticulés et alkylés partiellement ou totalement dérivant de polyaminoamides de formule générale :

⁅ CO-R₄-CO-z⁆ (XI)

dans laquelle R₄ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis primaire ou bis dérivé secondaire, et Z désigne un groupe d'une polyalkylène-polyamine bis-primaire, mono ou bis-secondaire et représente de préférence :
a) dans les proportions de 60 à 100 % en moles, le groupe 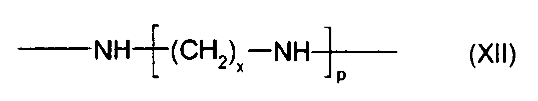 où x=2 et p=2 ou 3, ou bien x=3 et p=2
   ce groupe dérivant de la diéthylène-triamine, de la triéthylène-tétraamine ou de la dipropylène-triamine;
b) dans les proportions de 0 à 40 % en moles, le groupe (XII) ci-dessus, dans lequel x=2 et p=1 et qui dérive de l'éthylènediamine, ou le groupe dérivant de la pipérazine 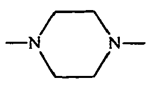
c) dans les proportions de 0 à 20 % en moles, le groupe -NH-(CH₂)₆-NH- dérivant de l'hexaméthylènediamine,
ces polyaminoamides étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane-sultone ou de leurs sels.

Les acides carboxyliques saturés sont choisis de préférence parmi les acides ayant 6 à 10 atomes de carbone tels que les acides adipique, 2,2,4-triméthyladipique et 2,4,4-triméthyladipique, téréphtalique, les acides à double liaison éthylénique comme, par exemple, les acides acrylique, méthacrylique, itaconique. Les alcane-sultones utilisées dans l'alcoylation sont de préférence la propane- ou la butane-sultone, les sels des agents d'alcoylation sont de préférence les sels de sodium ou de potassium.

(4) les polymères comportant des motifs zwittérioniques de formule : 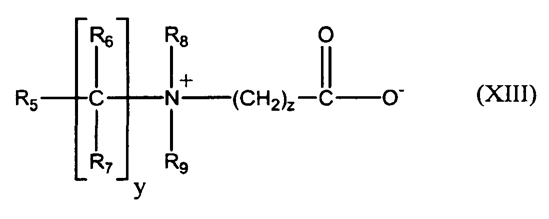 dans laquelle R₅ désigne un groupement insaturé polymérisable tel qu'un groupement acrylate, méthacrylate, acrylamide ou méthacrylamide, y et z représentent chacun un nombre entier de 1 à 3, R₆ et R₇ représentent un atome d'hydrogène, un groupement méthyle, éthyle ou propyle, R₈ et R₉ représentent un atome d'hydrogène ou un groupe alkyle de telle façon que la somme des atomes de carbone dans R₁₀ et R₁₁ ne dépasse pas 10.

Les polymères comprenant de tels motifs peuvent également comporter des motifs dérivés de monomères non zwittérioniques tels que l'acrylate ou le méthacrylate de diméthyl- ou diéthylaminoéthyle, ou des acrylates ou méthacrylates d'alkyle, des acrylamides ou méthacrylamides, ou l'acétate de vinyle.

(5) les polymères dérivés du chitosane comportant des motifs monomères répondant aux formules suivantes: 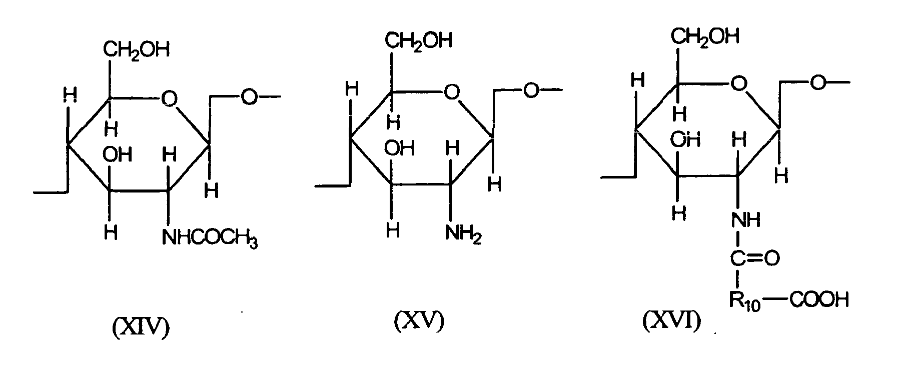 le motif (XIV) étant présent dans des proportions comprises entre 0 et 30 %, le motif (XV) dans des proportions comprises entre 5 et 50 % et le motif (XVI) dans des proportions comprises entre 30 et 90 %, étant entendu que dans ce motif F, R₁₀ représente un groupe de formule: 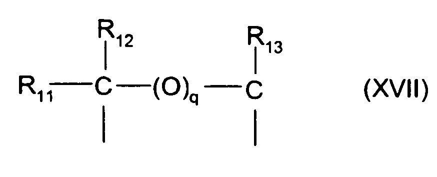 dans laquelle si q=0, R₁₁, R₁₂ et R₁₃, identiques ou différents, représentent chacun un atome d'hydrogène, un reste méthyle, hydroxyle, acétoxy ou amino, un reste monoalkylamine ou un reste dialkylamine éventuellement interrompus par un ou plusieurs atomes d'azote et/ou éventuellement substitués par un ou plusieurs groupes amine, hydroxyle, carboxyle, alkylthio, sulfonique, un reste alkylthio dont le groupe alkyle porte un reste amino, l'un au moins des groupes R₁₁, R₁₂ et R₁₃ étant dans ce cas un atome d'hydrogène ;
ou si q=1, R₁₁, R₁₂ et R₁₃ représentent chacun un atome d'hydrogène, ainsi que les sels formés par ces composés avec des bases
ou des acides.

(6) Les polymères dérivés de la N-carboxyalkylation du chitosane.

(7) Les polymères de motifs répondant à la formule générale (XVIII) décrits par exemple, dans le brevet français 1 400 366 : 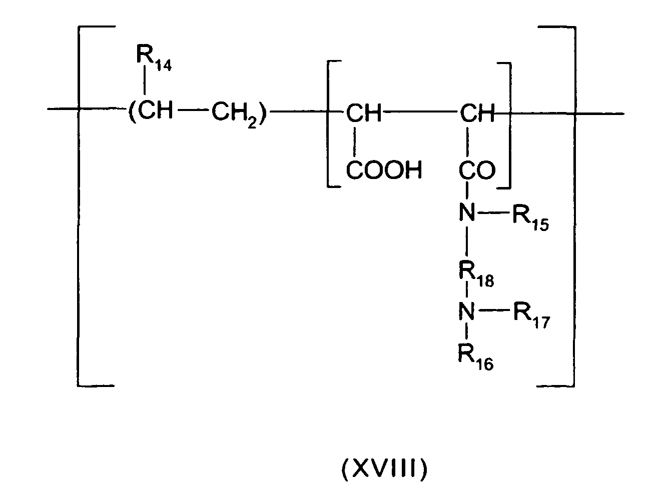 dans laquelle R₁₄ représente un atome d'hydrogène, un groupe CH₃O, CH₃CH₂O, phényle, R₁₅ désigne l'hydrogène ou un groupe alkyle inférieur tel que méthyle, éthyle, R₁₆ désigne l'hydrogène ou un groupe alkyle inférieur tel que méthyle, éthyle, R₁₇ désigne un groupe alkyle inférieur tel que méthyle, éthyle ou un groupe répondant à la formule: -R₁₈-N(R₁₆)₂, R₁₈ représentant un groupement -CH₂-CH₂-, -CH₂-CH₂-CH₂-, -CH₂-CH(CH₃)-, R₁₆ ayant les significations mentionnées ci-dessus,
ainsi que les homologues supérieurs de ces groupes et contenant jusqu'à 6 atomes de carbone.

(8) Des polymères amphotères du type -D-X-D-X- choisis parmi:
a) les polymères obtenus par action de l'acide chloracétique ou le chloracétate de sodium sur les composés comportant au moins un motif de formule:

   -D-X-D-X-D- (XIX)

   où D désigne un groupe 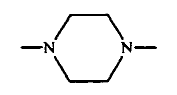 et X désigne le symbole E ou E', E ou E', identiques ou différents, désignent un groupe bivalent qui est un groupe alkylène à chaîne droite ou ramifiée comportant jusqu'à 7 atomes de carbone dans la chaîne principale non substituée ou substituée par des groupements hydroxyle, et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
b) Les polymères de formule :

   -D-X-D-X- (XX)
où D désigne un groupe 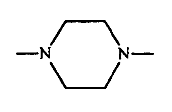 et X désigne le symbole E ou E' et au moins une fois E'; E ayant la signification indiquée ci-dessus et E' est un groupe bivalent qui est un groupe alkylène à chaîne droite ou ramifiée ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) Les copolymères alkyl(C₁-C₅)vinyléther/anhydride maléique modifié partiellement par semiamidification avec une N,N-dialkylaminoalkylamine telle que la N,N-diméthylaminopropylamine ou par semiestérification avec une N,N-dialcanolamine Ces copolymères peuvent également comporter d'autres comonomères vinyliques tels que le vinylcaprolactame.

Selon un mode de réalisation préféré de l'invention, les polymères amphotères fixant utilisables dans le procédé selon l'invention peuvent être choisis parmi les copolymères à blocs, ramifiés, comprenant :
(a) des motifs non ioniques dérivés d'au moins un monomère choisi parmi les (méth)acrylates d'alkyle en C₁-C₂₀, les N-mono-(alkyle en C₂-C₁₂)-(méth)acrylamides et les N,N-di-(alkyle en C₂-C₁₂)-(méth)acrylamide,
(b) des motifs anioniques dérivés d'au moins un monomère choisi parmi l'acide acrylique et l'acide méthacrylique, et
(c) des motifs polyfonctionnels dérivés d'au moins un monomère comportant au moins deux groupes fonctionnels insaturés polymérisables,
et ayant de préférence une structure constituée de blocs hydrophobes sur lesquels sont fixés, par l'intermédiaire des motifs polyfonctionnels (c), plusieurs blocs plus hydrophiles.

De préférence, les polymères amphotères présentent au moins deux températures de transition vitreuse (Tg) dont au moins une est supérieure à 20 °C et l'autre est inférieure à 20°C.

Les polymères amphotères préférés sont les polymères comportant des motifs dérivant :
a) d'au moins un monomère choisi parmi les acrylamides ou les méthacrylamides substitués sur l'azote par un groupe alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.

On peut citer en particulier les polymères vendus sous la dénomination AMPHOMER par la société NATIONAL STARCH.

Le milieu de la composition (B) contenant le polymère fixant est un milieu cosmétiquement acceptable aqueux, alcoolique ou hydroalcoolique. Les solvants organiques cosmétiquement acceptables sont de préférence des monoalcools, des polyols ou des éthers de ces alcools ou polyols. On peut citer par exemple l'éthanol, l'isopropanol, le glycérol, le propylèneglycol, l'éther monométhylique de propylèneglycol. L'éthanol est un solvant particulièrement préféré.

Généralement, le ou les polymères fixant représentent de 0,1 à 20 %, de préférence de 1 à 15 %, en poids du poids total de la composition (B).

L'étape de mise en forme du procédé selon la présente demande est une étape de coiffage au cours de laquelle on donne aux cheveux la forme désirée en utilisant simplement les mains ou bien des moyens mécaniques simples permettant de maintenir facilement en place des mèches de cheveux plus longues, tels que des pinces, peignes, barrettes, boudins et élastiques. Cette mise en place peut être réalisée par l'utilisateur lui-même et ne nécessite pas l'intervention d'un coiffeur. En tout état de cause cette étape ne comprend pas l'utilisation de moyens mécaniques de mise sous tension.

Cette étape est réalisée à température ambiante ou en chauffant à de manière à porter les cheveux à une température comprise entre 30 et 60°C.

Cette étape est généralement suivie d'un temps de pause allant de 10 minutes à 25 minutes.

A ce moment, il est alors possible de faire un rinçage. Il convient toutefois de veiller à ce que ce rinçage laisse une quantité suffisante de précipité pour conserver la forme de la coiffure lors de l'étape de fixation.

Généralement, l'étape de coiffage n'est pas suivie d'un rinçage.

La composition fixatrice (C) est appliquée de préférence sur les cheveux après le temps de pause suivant la mise en forme. Cette application se fait bien entendu dans la mesure du possible sans modification de la forme imposée à la chevelure dans l'étape précédente. Une telle application peut se faire par exemple à l'aide d'un dispositif aérosol ou d'un flacon-pompe dispensant une mousse fixatrice ou de fines gouttelettes d'une solution fixatrice. L'application de la composition fixatrice sous forme de mousse ou de fines gouttelettes permet un ramollissement du polymère précipité progressivement pendant l'étape de fixation. La nature de l'agent oxydant utilisé dans cette composition fixatrice n'est pas déterminante pour la présente invention et l'on peut utiliser n'importe quel agent oxydant connu utilisé habituellement pour l'oxydation des cheveux, tels que le peroxyde d'hydrogène, le peroxyde d'urée, les bromates, les persels et les mélanges de ceux-ci.

De préférence, l'agent oxydant est le peroxyde d'hydrogène.

Le temps de pause pour l'étape de fixation est généralement compris entre 2 et 30 minutes, de préférence entre 2 et 15 minutes et de manière encore plus préférée entre 2 et 7 minutes.

La composition restant sur les cheveux est ensuite éliminée par rinçage.

Avantageusement la composition fixatrice (C) contient au moins un tensio-actif, de préférence anionique.

Le rinçage est suivi éventuellement d'un lavage et/ou d'un autre traitement capillaire.

Avantageusement la composition réductrice (A) contient un polymère cationique. Elle peut aussi contenir un sel d'un acide dithio tel que le dithioglycolate d'ammonium.

Avantageusement l'étape d'application (a) d'une composition réductrice (A) est précédée d'une étape d'application d'une composition (O) contenant un polymère cationique.

Il est possible de faire suivre l'étape d'application d'un polymère cationique par un rinçage. Il convient toutefois de veiller à ce que ce rinçage laisse une quantité suffisante de polymère cationique pour que les propriétés apportées par ce polymère soient conservées.

Généralement, l'étape d'application d'un polymère cationique n'est pas suivie d'un rinçage.

En outre le dispositif à plusieurs compartiments peut contenir un compartiment (D1) comprenant une composition contenant un polymère cationique.

Au sens de la présente demande, "polymère cationique" désigne tout polymère contenant des groupements cationiques ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Une famille de polymères cationiques est celle des polymères cationiques siliconés. Parmi ces polymères, on peut citer :
(a) les polymères siliconés répondant à la formule (IV) suivante :

   R⁶ₐG⁵₃₋ₐ-Si(OSiG⁶₂)ₙ-(OSiG⁷_{b}R⁷_{2-b})ₘ-O-SiG⁸₃₋ₐ,-R⁸ₐ, (IV)

   dans laquelle :
   G⁵, G⁶, G⁷ et G⁸, identiques ou différents, désignent un atome d'hydrogène, un groupement phényle, OH, alkyle en C₁-C₁₈ par exemple méthyle, alcényle en C₂-C₁₈, ou alcoxy en C₁-C₁₈
   a, a', identiques ou différents, désignent le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R⁶, R⁷, R⁸, identiques ou différents, désignent un radical monovalent de formule -C_{q}H_{2q}Oₛ R⁹ₜL dans laquelle q est un nombre de 1 à 8, s et t, identiques ou différents, sont égaux à 0 ou à 1, R⁹ désigne un groupement alkylène éventuellement hydroxylé et L est un groupement aminé éventuellement quaternisé choisi parmi les groupements :
      -NR"-CH₂-CH₂-N'(R")₂
      -N(R")₂
      -N^{⊕}(R")₃ A⁻
      -N^{⊕}H(R")₂ A⁻
      -N^{⊕}H₂(R") A⁻
      -N(R")-CH₂-CH₂-N^{⊕}R" H₂ A⁻,
      dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.

Des produits correspondant à cette définition sont par exemple les polysiloxanes dénommés dans le dictionnaire CTFA "amodiméthicone" ou « triméthylsilylamodiméthicone ».

Un produit commercial répondant à cette définition est un mélange (90/10 en poids) d'un polydiméthylsiloxane à groupements aminoéthyl aminoisobutyle et d'un polydiméthylsiloxane commercialisé sous la dénomination Q2-8220 par la société DOW CORNING.

De tels polymères sont décrits par exemple dans la demande de brevet EP-A-95238.

Comme polymère de formule (IV) on peut citer les silicones aminées à groupement alcoxy comme la silicone WACKER BELSIL ADM LO 61.

D'autres polymères répondant à la formule (IV) sont les polymères siliconés répondant à la formule suivante (VI) :

T-(CH₂)ₛ-Si-[(OSiR⁵R⁶)ₜ-R⁷]_{y} (VI)

dans laquelle :
R₁₀ représente un radical hydrocarboné monovalent ayant de 1 à 18 atomes de carboné, et en particulier un radical alkyle en C₁-C₁₈, ou alcényle en C₂-C₁₈, par exemple méthyle ;
R₁₁ représente un radical hydrocarboné divalent, notamment un radical alkylène en C₁-C₁₈ ou un radical alkylèneoxy divalent en C₁-C₁₈, par exemple en C₁-C₈ ;
Q⁻ est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

De tels polymères sont décrits plus particulièrement dans le brevet US 4 185 087.

b- les composés de formule :

NH-[(CH₂)₃-Si[OSi(CH₃)₃]]₃

correspondant à la dénomination CTFA "aminobispropyldiméthicone".

Un polymère entrant dans cette classe est le polymère commercialisé par la Société Union Carbide sous la dénomination "Ucar Silicone ALE 56.

Lorsque ces polymères siliconés sont mis en oeuvre, une forme de réalisation particulièrement intéressante est leur utilisation conjointe avec des agents de surface cationiques et/ou non ioniques. On peut utiliser par exemple le produit commercialisé sous la dénomination "Emulsion Cationique DC 939" par la Société DOW CORNING qui comprend, outre l'amodiméthicone, des agents de surface cationiques et non ioniques.

Un autre produit commercial utilisable selon l'invention est le produit commercialisé sous la dénomination "Dow Corning Q2 7224" par la Société Dow Corning comportant en association un triméthylsilylamodiméthicone et des agents de surface non ioniques.

Les polymères du type polyamine, polyamidoamide, polyammonium quaternaire, utilisables conformément à la présente invention sont par exemple ceux décrits dans les brevets français n°2 505 348 ou 2 542 997. Parmi ces polymères, on peut citer :

(1) Les copolymères vinylpyrrolidone-acrylate ou - méthacrylate de dialkylamino-alkyle quaternisés ou non, tels que les produits commercialisés sous la dénomination "Gafquat®" par la Société ISP, comme par exemple Gafquat 734, 755 ou HS100 ou bien le produit dénommé "Copolymère 937". Ces polymères sont décrits en détail dans les brevets français 2 077 143 et 2 393 573.

(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.

(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthyl ammonium, de méthacrylmidopropyl triméthyl ammonium ou de diméthyl-diallylammonium.

Les produits commercialisés répondant à cette définition sont plus particulièrement les produits commercialisés sous la dénomination "Celquat L 200" et "Celquat H 100" par la Société National Starch.

(4) Les gommes de guar cationiques décrits plus particulièrement dans les brevets US 3 589 578 et 4 031 307 et plus particulièrement le produit commercialisé sous la dénomination "Jaguar C.13 S" commercialisé par la Société MEYHALL.

(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;

(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;

(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-dialcoylaminohydroxyalcoyldialcoylène triamine dans lesquels le radical alcoyle comporte de 1 à 4 atomes de carbone et désigne de préférence méthyle, éthyle, propyle. De tels polymères sont notamment décrits dans le brevet français 1.583.363.

Parmi ces dérivés, on peut citer plus particulièrement les polymères acide adipique/diméthylaminohydroxypropyl/diéthylène triamine commercialisés sous la dénomination "Cartaretine F, F4 ou F8" par la société Sandoz.

(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5:1 et 1,8:1. De tels polymères sont notamment décrits dans les brevets américains 3.227.615 et 2.961.347.

Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

(9) les cyclopolymères de méthyl diallyl amine ou de diallyl diméthyl ammonium tels que les homopolymères ou les copolymères comportant comme constituant principal de la chaîne des motifs répondant aux formules (VIII) ou (VIII') : 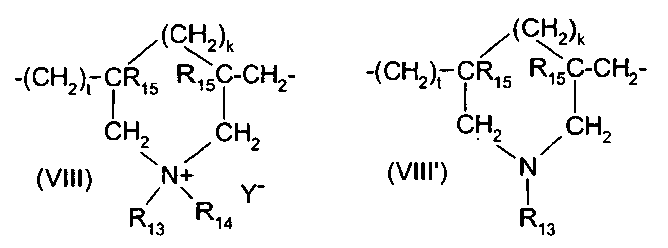 formules dans lesquelles k et t sont égaux à 0 ou 1, la somme k + t étant égale à 1 ; R₁₅ désigne un atome d'hydrogène ou un radical méthyle ; R₁₃ et R₁₄, indépendamment l'un de l'autre, désignent un groupement alkyle ayant de 1 à 8 atomes de carbone, un groupement hydroxyalkyle dans lequel le groupement alkyle a de préférence 1 à 5 atomes de carbone, un groupement amidoalkyle inférieur ou R₁₃ et R₁₄ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.

On peut citer par exemple l'homopolymère de chlorure de diallyldiméthylammonium commercialisé sous la dénomination "MERQUAT 100" par la société MERCK et les copolymères de chlorure de diallyldiméthylammonium et d'acrylamide commercialisés sous la dénomination "MERQUAT 550".

(10) le polymère de diammonium quaternaire contenant des motifs récurrents répondant à la formule (XIII) : 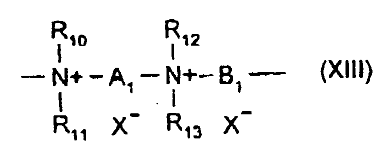 formule dans laquelle :
R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃, représentent un radical alkyle en C₁-C₆ linéaire ou ramifié substitué par un groupement nitrile, ester, acyle, amide ou -CO-O-R₂₀-D ou -CO-NH-R₂₀-D où R₂₀ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R₁₀ et R₁₂ peuvent former avec les deux atomes d'azote auxquels ils sont rattachés un cycle pipérazinique ; en outre si A1 désigne un radical alkylène ou hydroxyalkylène, linéaire ou ramifié, saturé ou insaturé, B1 peut également désigner un groupement (CH₂)ₙ-CO-D-OC-(CH₂)ₙ-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH₂-CH₂-O)ₓ-CH₂-CH₂-

      -[CH₂-CH(CH₃)-O]_{y}-CH₂-CH(CH₃)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH₂-CH₂-S-S-CH₂-CH₂- ;
   d) un groupement uréylène de formule : -NH-CO-NH- ;

De préférence, X⁻ est un anion tel que le chlorure ou le bromure.

Ces polymères ont une masse moléculaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut plus particulièrement utiliser les polymères qui sont constitués de motifs récurrents répondant à la formule :

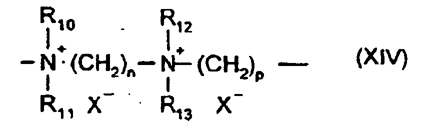

Dans laquelle R₁₀, R₁₁, R₁₂ et R₁₃, identiques ou différents, désignent un radical alkyle ou hydroxyalkyle ayant de 1 à 4 atomes de carbones environ, n et p sont des nombres entiers variant de 2 à 20 et X- est un anion dérivé d'un acide minéral ou organique.

Un composé particulièrement préféré est celui pour lequel R₁₀, R₁₁, R₁₂ et R₁₃ représentent un radical méthyle et n=3, p = 6 et X- est un ion chlorure, dénommé Hexadiméthrine Chloride selon la nomenclature INCI (CTFA) tel que le Mexomere® PO de la société Chimex.

(11) les polymères constitués de motifs récurrents polyammonium quaternaires par exemple constitués de motifs de formule (X) : 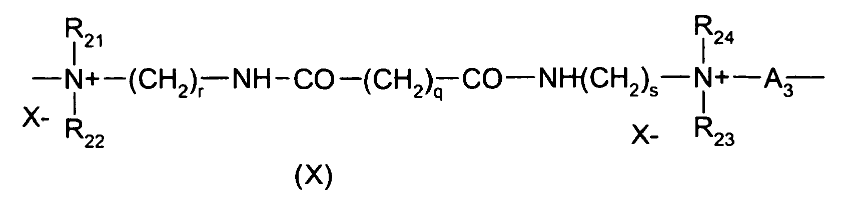 formule dans laquelle :
R₂₁, R₂₂, R₂₃ et R₂₄, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
où p est égal à 0 ou à un nombre entier compris entre 1 et 6, sous réserve que R₂₁, R₂₂, R₂₃ et R₂₄ ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A₃ désigne un radical d'un dihalogénure ou représente de préférence -CH₂-CH₂-O-CH₂-CH₂-.

De tels composés sont notamment décrits dans la demande de brevet EP-A-122 324.

On peut par exemple citer parmi ceux-ci, les produits "Mirapol® A 15", "Mirapol® AD1", "Mirapol® AZ1" et "Mirapol® 175" commercialisés par la société Miranol.

(12) les homopolymères ou copolymères dérivés des acides acrylique ou méthacrylique et comportant des motifs : 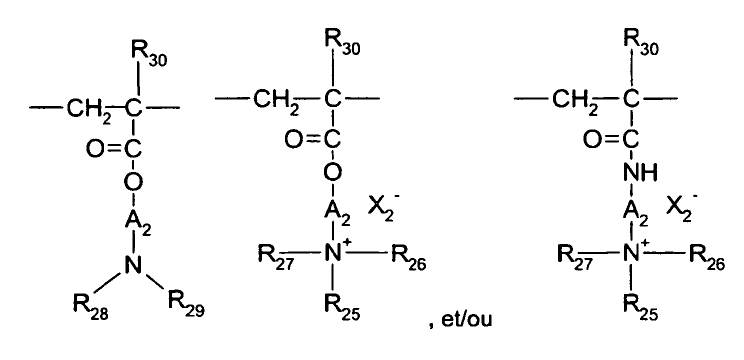 dans lesquels les groupements R₃₀ désignent indépendamment H ou CH₃,
les groupements A₂ désignent indépendamment un groupe alcoyle linéaire ou ramifié de 1 à 6 atomes de carbone ou un groupe hydroxyalcoyle de 1 à 4 atomes de carbone,
les groupements R₂₅, R₂₆, R₂₇, identiques ou différents, désignant indépendamment un groupe alcoyle de 1 à 18 atomes de carbone ou un radical benzyle,
les groupements R₂₈ et R₂₉ représentent un atome d'hydrogène ou un groupement alcoyle de 1 à 6 atomes de carbone,
X₂⁻ désigne un anion, par exemple méthosulfate ou halogénure, tel que chlorure ou bromure.

Le ou les comonomères utilisables dans la préparation des copolymères correspondants appartiennent à la famille des acrylamides, méthacrylamides, diacétone acrylamides, acrylamides et méthacrylamides substitués à l'azote par des alcoyle inférieurs, des esters d'alcoyles, des acides acrylique ou méthacrylique, la vinylpyrrolidone ou des esters vinyliques.

(13) Les polymères quaternaires de vinylpyrrolidone et de vinylimidazole tels que par exemple les produits commercialisés sous les dénominations LUVIQUAT® FC 905, FC 550 et FC 370 par la société BASF.

(14) Les polyamines comme le Polyquart H commercialisé par HENKEL, référencé sous le nom de « POLYETHYLENEGLYCOL (15) TALLOW POLYAMINE » dans le dictionnaire CTFA.

(15) Les polymères réticulés de chlorure de méthacryloyloxyéthyl triméthyl ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale. Cette dispersion est commercialisée sous le nom de « SALCARE® SC 95 » par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les cyclopolymères, en particulier les homolymères du chlorure de diméthyldiallylammonium commercialisé sous la dénomination « MERQUAT® 100 » par la Société MERCK, les polymères de diammonium quaternaire de formule (IX) ou de formule (X).

Selon l'invention, le ou les polymères cationiques peuvent représenter de 0,01% à 20 % en poids, de préférence de 0,1 % à 15 % en poids, et encore plus préférentiellement de 0,5 % à 5 % en poids, du poids total de la composition (O) ou de la composition (A).

Chacune des compositions du dispositif à plusieurs compartiments peut contenir en outre des principes actifs et adjuvants cosmétiques utilisés couramment dans le domaine capillaire. Ces additifs sont choisis par exemple parmi les vitamines, les acides aminés, les oligopeptides, les peptides, les protéines hydrolysées ou non, modifiées ou non, les enzymes, les acides et alcools gras ramifiés ou non, les cires animales, végétales ou minérales, les céramides et les pseudo-céramides, les acides organiques hydroxylés, les filtres UV, les agents anti-oxydants et les agents anti-radicaux libres, les agents chélatants, les agents antipelliculaires, les agents régulateurs de séborrhée, les agents apaisants, les agents tensioactifs cationiques, anioniques, non-ioniques ou amphotères, les polymères non ioniques, les silicones non ioniques, les huiles minérales, végétales ou animales, les polyisobutènes et poly(α-oléfines), les esters gras, les agents colorants capillaires tels que les colorants directs et les pigments.

Bien entendu, l'homme de l'art veillera à choisir ce ou ces principes actifs et adjuvants cosmétiques complémentaires de manière telle que les propriétés avantageuses attachées intrinsèquement au dispositif et procédé conformes à l'invention ne soient pas, ou substantiellement pas, altérées par la ou les adjonctions envisagées.

Les exemples qui suivent servent à illustrer l'invention sans toutefois présenter un caractère limitatif.

### EXEMPLES

On utilise les compositions suivantes :

| Composition réductrice (A) | |
|---|---|
| Acide thioglycolique | 5,0 g |
| Ammoniaque | 6,0 g |
| Chlorure d'ammonium | 5,0 g |
| Hydroxypropylguar | 1,0 g |
| Mexomere® PO* (Chimex) | 1,5 g |
| Tégobétaine® HS** (Goldschmidt) | 1,5 g |
| Parfum, peptisant q.s. | |
| Eau déminéralisée q.s.p. | 100 g |

| Composition (B) | |
|---|---|
| Luviset Si-P.U.R.A | 5,13 g |
| AQ 48 | 6,15 g |
| Parfum | 0,40g |
| Alcool | 28, 95 g |
| Eau déminéralisée q.s.p. | 100 g |

| | |
|---|---|
| * polymère de N,N,N,N-tétraméthylhexaméthylènediamine et de chlorure de triméthylène (INCI : Hexadimethrine Chloride) ** cocamidopropylbétaïne et laurate de glycérol | |

Aérosol contenant 65 g de composition (B) et 35 g de propulseur diméthyléther.

| Composition fixatrice (C) | |
|---|---|
| Peroxyde d'hydrogène en solution à 50 % | 4,8 g |
| Stabilisants | 0,03 g |
| Laurylsulfate d'ammonium | 4,0 g |
| Parfum, peptisant q.s. | |
| Eau déminéralisée q.s.p. | 100 g |

Ces trois compositions ont été appliquées conformément au procédé selon la présente demande sur des demi-têtes.

A titre de comparatif, l'autre moitié de chaque demi-tête a été simplement rincée à l'eau. Les cheveux des demi-têtes traitées selon le procédé conforme à l'invention ont été jugés plus modelables, la chevelure a été jugée plus volumineuse : le procédé permet un meilleur coiffant avec un apport de texture et de la masse.

Lorsque les cheveux des demi-têtes étaient des cheveux colorés, on observe un respect de la coloration, ainsi qu'une rémanence de ces effets dans le temps.

## Revendications

1. Procédé de déformation permanente des fibres kératiniques, de préférence les fibres kératiniques humaines et en particulier les cheveux, comprenant les étapes suivantes, dans l'ordre suivant :
(a) application sur les fibres kératiniques d'une composition réductrice (A) contenant un agent réducteur des fibres kératiniques et au moins un sel organique ou minéral,
(b) mise en forme des fibres kératiniques par coiffage, et application sur les fibres kératiniques d'une composition (B) comprenant au moins un polymère fixant choisi parmi les polymères fixant anioniques et amphotères, dans un milieu aqueux, alcoolique ou hydro-alcoolique,
(c) après un temps de pause, application sur les fibres kératiniques d'une composition fixatrice (C) contenant un agent oxydant,
(d) après un temps de pause, rinçage des fibres kératiniques ;
les sous-étapes de mise en forme des fibres kératiniques par coiffage et application sur les fibres kératiniques d'une composition (B) de l'étape (b) étant réalisées dans un des ordres suivants : mise en forme des fibres kératiniques par coiffage puis application sur les fibres kératiniques d'une composition (B) ; application sur les fibres kératiniques d'une composition (B) puis mise en forme des fibres kératiniques par coiffage ; ou mise en forme des fibres kératiniques par coiffage et simultanément application sur les fibres kératiniques d'une composition (B).

2. Procédé de déformation permanente selon la revendication 1 **caractérisé en ce que** l'étape (b) est suivie d'un rinçage.

3. Procédé de déformation permanente selon l'une quelconque des revendications précédentes **caractérisé en ce que** les sels organiques ou minéraux sont choisis parmi les sels d'ammonium et les sels de métaux alcalins.

4. Procédé de déformation permanente selon la revendication précédente **caractérisé par le fait que** les sels d'ammonium et/ou les sels de métaux alcalins sont apportés par l'agent réducteur.

5. Procédé de déformation permanente selon l'une quelconque des revendications précédentes **caractérisé par le fait que** la concentration des sels organiques ou minéraux dans la composition réductrice est supérieure à 0,3 M, de préférence comprise entre 0,5 et 6 M.

6. Procédé de déformation permanente selon la revendication précédente **caractérisé en ce que** les sels organiques ou minéraux sont choisis parmi le chlorure de sodium, le chlorure de magnésium, le carbonate d'ammonium et le citrate d'ammonium.

7. Procédé de déformation permanente selon l'une des revendications précédentes **caractérisé par le fait que** l'agent réducteur est choisi parmi les sulfites, les bisulfites et les thiols.

8. Procédé de déformation permanente selon la revendication précédente **caractérisé par le fait que** l'agent réducteur est choisi parmi la cystéine, la cystéamine, l'acide thiolactique et l'acide thioglycolique et les sels cosmétiquement acceptables de ceux-ci, en particulier le thioglycolate d'ammonium.

9. Procédé de déformation permanente selon l'une des revendications précédentes **caractérisé par le fait que** la quantité d'agent réducteur dans la composition réductrice (a) va de 1 % à 25 % en poids, de préférence de 1 à 10 % en poids par rapport au poids total de la composition.

10. Procédé de déformation permanente selon l'une quelconque des revendications précédentes **caractérisé en ce que** le polymère fixant anionique est choisi parmi les copolymères d'acide acrylique ; les copolymères dérivés d'acide crotonique ; les copolymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; les polyuréthanes anioniques élastomères ou non ; les polymères à groupements sulfoisophtalates et les polymères siliconés greffés anioniques.

11. Procédé de déformation permanente selon l'une quelconque des revendications précédentes **caractérisé en ce que** le polymère fixant amphotère est choisi parmi les copolymères comportant des motifs dérivant :
• d'au moins un monomère choisi parmi les acrylamides ou les méthacrylamides substitués sur l'azote par un groupe alkyle,
• d'au moins un comonomère acide contenant un ou plusieurs groupements carboxyliques réactifs, et
• d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.

12. Procédé de déformation permanente selon l'une quelconque des revendications précédentes **caractérisé en ce que** le ou les polymères fixant sont présents en une quantité allant de 0,1 à 20%, de préférence de 1 à 15% en poids par rapport au poids total de la composition (B).

13. Procédé de déformation permanente selon l'une quelconque des revendications précédentes **caractérisé en ce que** l'agent oxydant est choisi parmi le peroxyde d'hydrogène, le peroxyde d'urée, les bromates, les persels et les mélanges de ceux-ci.

14. Procédé de déformation permanente selon la revendication précédente **caractérisé par le fait que** le temps de pose pour l'étape de fixation est généralement compris entre 2 et 30 minutes, de préférence entre 2 et 15 minutes et de manière encore plus préférée entre 2 et 7 minutes.

15. Procédé de déformation permanente selon l'une quelconque des revendications précédentes **caractérisé en ce que** la composition fixatrice contient au moins un tensio-actif, de préférence anionique.

16. Procédé de déformation permanente selon l'une quelconque des revendications précédentes **caractérisé en ce que** la composition réductrice (A) contient un polymère cationique.

17. Procédé de déformation permanente selon l'une quelconque des revendications précédentes **caractérisé en ce que** l'étape d'application d'une composition réductrice (a) est précédée d'une étape d'application d'une composition contenant un polymère cationique.

18. Procédé de déformation permanente selon la revendication 17 **caractérisé en ce que** l'étape d'application d'une composition comprenant au moins un polymère cationique est suivie d'un rinçage

19. Procédé de déformation permanente selon l'une quelconque des revendications 16 à 18 **caractérisé en ce que** le polymère cationique est choisi parmi les dérivés d'éther de cellulose quaternaires, les copolymères de cellulose avec un monomère hydrosoluble d'ammonium quaternaire, les cyclopolymères, les polysaccharides cationiques, les polymères cationiques siliconés, les copolymères vinylpyrrolidone-acrylate ou -méthacrylate de dialkylamino-alkyle quaternisés ou non, les polymères quaternaires de vinylpyrrolidone et de vinylimidazole, les polyamidoamines, les polymères constitués de motifs récurrent polyammonium quaternaires et leurs mélanges.

## Claims

1. Process for permanently reshaping keratin fibres, preferably human keratin fibres and in particular the hair, including the following steps, in the following order:
(a) application to the keratin fibres of a reducing composition (A) containing a keratin fibre reducing agent and at least one organic or mineral salt,
(b) shaping of the keratin fibres by styling and application to the keratin fibres of a composition (B) comprising at least one fixing polymer chosen from anionic and amphoteric fixing polymers, in an aqueous, alcoholic or aqueous-alcoholic medium,
(c) after a leave-on time, application to the keratin fibres of a fixing composition (C) containing an oxidizing agent,
(d) after a leave-on time, rinsing of the keratin fibres;
the sub-steps of shaping of the keratin fibres by styling and application to the keratin fibres of a composition (B) of step (b) being performed in one of the following orders: shaping of the keratin fibres by styling, followed by application to the keratin fibres of a composition (B); application to the keratin fibres of a composition (B), followed by shaping of the keratin fibres by styling; or shaping of the keratin fibres by styling and simultaneous application to the keratin fibres of a composition (B).

2. Permanent reshaping process according to Claim 1, **characterized in that** step (b) is followed by rinsing.

3. Permanent reshaping process according to either of the preceding claims, **characterized in that** the organic or mineral salts are chosen from ammonium salts and alkali metal salts.

4. Permanent reshaping process according to the preceding claim, **characterized in that** the ammonium salts and/or the alkali metal salts are provided by the reducing agent.

5. Permanent reshaping process according to any one of the preceding claims, **characterized in that** the concentration of the organic or mineral salts in the reducing composition is greater than 0.3 M and preferably between 0.5 and 6 M.

6. Permanent reshaping process according to the preceding claim, **characterized in that** the organic or mineral salts are chosen from sodium chloride, magnesium chloride, ammonium carbonate and ammonium citrate.

7. Permanent reshaping process according to one of the preceding claims, **characterized in that** the reducing agent is chosen from sulfites, bisulfites and thiols.

8. Permanent reshaping process according to the preceding claim, **characterized in that** the reducing agent is chosen from cysteine, cysteamine, thiolactic acid and thioglycolic acid and cosmetically acceptable salts thereof, in particular ammonium thioglycolate.

9. Permanent reshaping process according to one of the preceding claims, **characterized in that** the amount of reducing agent in the reducing composition (A) ranges from 1% to 25% by weight and preferably from 1% to 10% by weight relative to the total weight of the composition.

10. Permanent reshaping process according to any one of the preceding claims, **characterized in that** the anionic fixing polymer is chosen from acrylic acid copolymers; crotonic acid-based copolymers; copolymers derived from maleic, fumaric or itaconic acid or anhydride with vinyl esters, vinyl ethers, vinyl halides or phenylvinyl derivatives, acrylic acid and esters thereof; elastomeric or non-elastomeric anionic polyurethanes; polymers containing sulfoisophthalate groups and anionic grafted silicone polymers.

11. Permanent reshaping process according to any one of the preceding claims, **characterized in that** the amphoteric fixing polymer is chosen from copolymers comprising units derived from:
• at least one monomer chosen from acrylamides and methacrylamides substituted on the nitrogen with an alkyl group,
• at least one acidic comonomer containing one or more reactive carboxylic groups, and
• at least one basic comonomer such as acrylic and methacrylic acid esters containing primary, secondary, tertiary and quaternary amine substituents, and the product of quaternization of dimethylaminoethyl methacrylate with dimethyl or diethyl sulfate.

12. Permanent reshaping process according to any one of the preceding claims, **characterized in that** the fixing polymer(s) is (are) present in an amount ranging from 0.1% to 20% and preferably from 1% to 15% by weight relative to the total weight of composition (B).

13. Permanent reshaping process according to any one of the preceding claims, **characterized in that** the oxidizing agent is chosen from hydrogen peroxide, urea peroxide, bromates and persalts, and mixtures thereof.

14. Permanent reshaping process according to the preceding claim, **characterized in that** the leave-on time for the fixing step is generally between 2 and 30 minutes, preferably between 2 and 15 minutes and even more preferably between 2 and 7 minutes.

15. Permanent reshaping process according to any one of the preceding claims, **characterized in that** the fixing composition contains at least one surfactant, which is preferably anionic.

16. Permanent reshaping process according to any one of the preceding claims, **characterized in that** the reducing composition (A) contains a cationic polymer.

17. Permanent reshaping process according to any one of the preceding claims, **characterized in that** the step of applying a reducing composition (A) is preceded by a step of applying a composition containing a cationic polymer.

18. Permanent reshaping process according to Claim 17, **characterized in that** the step of applying a composition comprising at least one cationic polymer is followed by rinsing.

19. Permanent reshaping process according to any one of Claims 16 to 18, **characterized in that** the cationic polymer is chosen from quaternary cellulose ether derivatives, copolymers of cellulose with a watersoluble quaternary ammonium monomer, cyclopolymers, cationic polysaccharides, cationic silicone polymers, quaternized or non-quaternized vinylpyrrolidone/dialkylaminoalkyl acrylate or methacrylate copolymers, quaternary polymers of vinylpyrrolidone and of vinylimidazole, polyamidoamines, and polymers consisting of polyquaternary ammonium repeating units, and mixtures thereof.

## Patentansprüche

1. Verfahren zur dauerhaften Verformung von Keratinfasern, vorzugsweise menschlichen Keratinfasern und insbesondere des Haars, das die folgenden Schritte in der folgenden Reihenfolge umfasst:
(a) Aufbringen einer reduzierenden Zusammensetzung (A), die ein Reduktionsmittel für Keratinfasern und mindestens ein organisches oder anorganisches Salz enthält, auf die Keratinfasern,
(b) Formen der Keratinfasern durch Frisieren und Aufbringen einer Zusammensetzung (B), die mindestens ein fixierendes Polymer, ausgewählt aus der Reihe der anionischen und amphoteren fixierenden Polymere, in einem wässrigen, alkoholischen oder wässrigalkoholischen Medium umfasst, auf die Keratinfasern,
(c) nach einem Zeitabstand Aufbringen einer fixierenden Zusammensetzung (C), die ein Oxidationsmittel enthält, auf die Keratinfasern,
(d) nach einem Zeitabstand Spülen der Keratinfasern; wobei die Unterschritte des Formens der Keratinfasern durch Frisieren und Ausbringen einer Zusammensetzung (B) von Schritt (b) auf die Keratinfasern in einer der folgenden Reihenfolgen erfolgen: Formen der Keratinfasern durch Frisieren und anschließendes Aufbringen einer Zusammensetzung (B) auf die Keratinfasern; Aufbringen einer Zusammensetzung (B) auf die Keratinfasern und anschließendes Formen der Keratinfasern durch Frisieren, oder Formen der Keratinfasern durch Frisieren und gleichzeitiges Aufbringen einer Zusammensetzung (B) auf die Keratinfasern.

2. Verfahren zur dauerhaften Verformung nach Anspruch 1, **dadurch gekennzeichnet, dass** nach Schritt (b) ein Spülen erfolgt.

3. Verfahren zur dauerhaften Verformung nach einem der vorhergehenden Ansprüche, **dadurch gekennzeichnet, dass** die organischen oder anorganischen Salze aus der Reihe der Ammoniumsalze und der Alkalimetallsalze ausgewählt werden.

4. Verfahren zur dauerhaften Verformung nach dem vorhergehenden Anspruch, **dadurch gekennzeichnet, dass** die Ammoniumsalze und/oder die Alkalimetallsalze von dem Reduktionsmittel bereitgestellt werden.

5. Verfahren zur dauerhaften Verformung nach einem der vorhergehenden Ansprüche, **dadurch gekennzeichnet, dass** die Konzentration der organischen oder anorganischen Salze in der reduzierenden Zusammensetzung über 0,3 M, vorzugsweise zwischen 0,5 und 6 M, beträgt.

6. Verfahren zur dauerhaften Verformung nach dem vorhergehenden Anspruch, **dadurch gekennzeichnet, dass** die organischen oder anorganischen Salze aus der Reihe Natriumchlorid, Magnesiumchlorid, Ammoniumcarbonat und Ammoniumcitrat ausgewählt werden.

7. Verfahren zur dauerhaften Verformung nach einem der vorhergehenden Ansprüche, **dadurch gekennzeichnet, dass** das Reduktionsmittel aus der Reihe der Sulfite, Bisulfite und Thiole ausgewählt wird.

8. Verfahren zur dauerhaften Verformung nach dem vorhergehenden Anspruch, **dadurch gekennzeichnet, dass** das Reduktionsmittel aus der Reihe Cystein, Cysteamin, Thiomilchsäure und Thioglykolsäure und deren kosmetisch unbedenklichen Salzen, insbesondere Ammoniumthioglykolat, ausgewählt wird.

9. Verfahren zur dauerhaften Verformung nach einem der vorhergehenden Ansprüche, **dadurch gekennzeichnet, dass** die Menge an Reduktionsmittel in der reduzierenden Zusammensetzung (A) 1 Gew.-% bis 25 Gew.-%, vorzugsweise 1 bis 10 Gew.-%, in Bezug auf das Gesamtgewicht der Zusammensetzung ausmacht.

10. Verfahren zur dauerhaften Verformung nach einem der vorhergehenden Ansprüche, **dadurch gekennzeichnet, dass** das anionische fixierende Polymer aus der Reihe der Acrylsäurecopolymere, der von Crotonsäure abgeleiteten Copolymere; der von Maleinsäure, Fumarsäure, Itaconsäure oder ihren Anhydriden und Vinylestern, Vinylethern, Vinylhalogeniden, Phenylvinylderivaten, Acrylsäure und ihren Estern abgeleiteten Copolymere; den gegebenenfalls elastomeren anionischen Polyurethanen; den Polymeren mit Sulfoisophthalatgruppen und den anionisch gepfropften Siliconpolymeren ausgewählt wird.

11. Verfahren zur dauerhaften Verformung nach einem der vorhergehenden Ansprüche, **dadurch gekennzeichnet, dass** das amphotere fixierende Polymer aus der Reihe der Copolymere mit Motiven, die sich von folgenden ableiten, ausgewählt wird:
• von mindestens einem Monomer aus der Reihe der am Stickstoff durch eine Alkylgruppe substituierten Acrylamide oder Methacrylamide,
• von mindestens einem sauren Comonomer, das eine oder mehrere reaktionsfähige Carboxylgruppen enthält, und
• von mindestens einem basischen Comonomer, wie Acrylsäure- und Methacrylsäureestern mit primären, sekundären, tertiären und quartären Aminsubstituenten, und dem Quartärnisierungsprodukt von Dimethylaminoethylmethacrylat mit Dimethyl- oder Diethylsulfat.

12. Verfahren zur dauerhaften Verformung nach einem der vorhergehenden Ansprüche, **dadurch gekennzeichnet, dass** das oder die fixierende(n) Polymer(e) in einer Menge von 0,1 bis 20 Ges.-%, vorzugsweise von 1 bis 15 Gew.-% in Bezug auf das Gesamtgewicht der Zusammensetzung (B) vorliegen.

13. Verfahren zur dauerhaften Verformung nach einem der vorhergehenden Ansprüche, **dadurch gekennzeichnet, dass** das Oxidationsmittel aus der Reihe Wasserstoffperoxid, Harnstoffperoxid, Bromate, Persalze und ihren Mischungen ausgewählt wird.

14. Verfahren zur dauerhaften Verformung nach dem vorhergehenden Anspruch, **dadurch gekennzeichnet, dass** der Zeitabstand für den Fixierschritt im Allgemeinen zwischen 2 und 30 Minuten, vorzugsweise zwischen 2 und 15 Minuten und noch stärker bevorzugt zwischen 2 und 7 Minuten beträgt.

15. Verfahren zur dauerhaften Verformung nach einem der vorhergehenden Ansprüche, **dadurch gekennzeichnet, dass** die fixierende Zusammensetzung mindestens ein Tensid, vorzugsweise ein anionisches Tensid, enthält.

16. Verfahren zur dauerhaften Verformung nach einem der vorhergehenden Ansprüche, **dadurch gekennzeichnet, dass** die reduzierende Zusammensetzung (A) ein kationisches Polymer enthält.

17. Verfahren zur dauerhaften Verformung nach einem der vorhergehenden Ansprüche, **dadurch gekennzeichnet, dass** vor dem Schritt des Aufbringens einer reduzierenden Zusammensetzung (A) ein Schritt des Aufbringens einer Zusammensetzung, die ein kationisches Polymer enthält, erfolgt.

18. Verfahren zur dauerhaften Verformung nach Anspruch 17, **dadurch gekennzeichnet, dass** nach dem Schritt des Aufbringens einer Zusammensetzung, die mindestens ein kationisches Polymer umfasst, ein Spülen erfolgt.

19. Verfahren zur dauerhaften Verformung nach einem der Ansprüche 16 bis 18, **dadurch gekennzeichnet, dass** das kationische Polymer aus der Reihe quartäre Celluloseetherderivate, Copolymere der Cellulose mit einem wasserlöslichen quartären Ammoniummonomer, der Cyclopolymere, der kationischen Polysaccharide, der silikonhaltigen kationischen Polymere, der gegebenenfalls quartärnisierten Dialkylaminoalkyl-Vinylpyrrolidon-Acrylat- oder -Methacrylat-Copolymere, der quartären Vinylpyrrolidon- und Vinylimidazolpolymere, der Polyamidoamine, der aus quartären Polyammoniumwiederholungseinheiten bestehenden Polymere und ihren Mischungen ausgewählt wird.
